# EUROPEAN PATENT SPECIFICATION

(11) **EP 2 327 378 B1**
(45) Date of publication and mention of the grant of the patent: **03.06.2015**
(21) Application number: 10425358.8
(22) Date of filing: 23.11.2010
(51) Int. Cl.: A61F 2/78

(54) **A cap for a limb prosthesis and a prosthesis comprising said cap**
Kappe für eine Gliedmaßenprothese und Prothese, die diese Kappe umfasst
Capuchon pour prothèse de membre et prothèse comprenant ledit capuchon

(30) Priority: 25.11.2009 IT FI20090247
(43) Date of publication of application: 01.06.2011
(73) Proprietor: Pieraccini, Amedeo, 52100 Arezzo (IT)
(72) Inventor: Pieraccini, Amedeo, 52100 Arezzo (IT)
(74) Representative: Mannucci, Michele

(56) References cited:
- US-A- 4 923 475
- US-A- 5 156 629
- US-A- 5 405 405
- US-B1- 6 899 737

## Description

### Technical field

The present invention relates to improvements to prostheses for limbs, in particular for the lower limbs, and to accessories for said prostheses.

### Prior art

Prostheses for limbs, in particular prostheses for the lower limbs, have several drawbacks in particular as regards the system for connecting the limb stump.

Both exoskeletal and endoskeletal prostheses for the lower limb are interfaced to the limb stump through a so-called socket, that is, a portion of the prosthesis shaped as a concave seat wherein the stump is inserted and retained therein to allow walking. In the simplest and most rudimental prostheses the stump is directly inserted into the socket that is made of a substantially stuff material, for example wooden or, more recently, a synthetic resin optionally reinforced with carbon fibre.

In more advanced prostheses, a so-called cap is interposed between the inner surface of the socket and the stump, which adheres to the stump and forms a sort of intermediate layer between the stump and the actual socket. This cap is normally made of silicone materials or other flexible materials that allow the movements of the stump muscles. In some prostheses there is also provided a vacuum system that by the effect of the walking movement, generates suction between the cap and the inner surface of the socket, that is, a certain degree of vacuum, which serves for retaining the artificial limb or prosthesis adhering to the stump by a sort of suction cup effect.

Despite the improvements made by the presence of the flexible cap interposed between the stump and the socket, the prostheses exhibit several drawbacks. In the first place, the stump, as a normal limb, tends to change in volume even during the day. Despite the interposition of the flexible cap between the stump and the stiff socket, these variations in volume imply several drawbacks for the person wearing the prosthesis. If the stump volume increases beyond the volume the prosthesis has been sized for, an excessive constriction of the stump may occur, with difficulties to blood circulation up to necrosis phenomena in the most serious cases. On the other hand, when the stump volume is excessively reduced compared to that the prosthesis has been sized for, the adhesion is lost and the patient is at risk of losing the prosthesis or in any case he/she encounters serious walking difficulties.

Moreover, despite the presence of the flexible cap material, when the stump volume decreases the latter penetrates too much into the socket and presses with the lower bony portion against the socket bottom, creating tissue squeezing points with consequent pain and risk of damaging the tissues. In the case of transfemoral prostheses, the excessive penetration of the stump into the socket may cause the interference between the upper socket edge and the patient's groin, with the risk of damaging the tissues in the groin area.

A cap according to the preamble of claim 1 is known from US 5 156 629 or US 4 923 475.

The suction systems mentioned above may in some cases worsen the situation creating an excessive interference between stump and socket.

### Summary of the invention

The object of the present invention is to provide a prosthesis system that alleviates at least partly one or more of the drawbacks of the traditional systems.

Substantially, according to one aspect, the invention provides a cap for the application of a limb prosthesis (in particular a lower limb prosthesis), intended for being applied on the limb stump and for forming an interface between the stump and the prosthesis socket, which comprises a dual membrane, i.e. a dual layer, structure, defining a hollow space or intermediate air chamber. The inner surface of a first inner membrane forms a concavity wherein the stump is inserted. A second membrane arranged outside the first one defines the outer surface of the cap that contacts and adheres to the socket. Said hollow space or airtight chamber is defined between the two membranes for the purposes described below.

A cap thus made may be easily customized and adjusted to the single patient, avoiding the onset of pressures, frictions or other stresses located in sensitive points for the growth of neuromas or the like, without causing an increase in production costs.

The cap according to the invention is particularly effective in the step immediately subsequent the amputation of the limb for a period of 2-3 years, during which there has not yet been a complete stabilization of the tissues of the amputated limb. It is also particularly effective for subjects with vascular problems and is recommended to those that want to make a physical or agonistic activity.

In practice, the first membrane and the second membrane may be shaped as a cap, that is, they both define an inner volume, the first whereof - formed by the first membrane - defines the seat of insertion of the stump and the second one - formed by the outer membrane - defines the zone for seating the first membrane. The two membranes are preferably welded along a common upper edge. In some embodiments, there may also be provided a discontinuous welding, or a welding in portions, between the two membranes in a lower zone, close to the bottom of the same membranes, where a reservoir of fluid under pressure is defined. For example, a welding may be provided, interrupted by channels or communication conduits between the two hollow space portions that form above and under the welding. Preferably, the lower zone of the outer membrane has a larger thickness than the upper or central portion. Typically, the lower portion of the outer membrane may have a thickness of two or four times, preferably two to three times larger than the median zone. In this way, the hollow space is divided in at least an upper chamber and a lower chamber. During use, the lower chamber is adjacent the bottom of the prosthesis socket. It forms a sort of shock absorber or air bearing between the distal end of the stump and the socket bottom, increasing the user's comfort. Moreover, the larger thickness of the outer membrane prevents it from swelling or in any case limits the expansion thereof, so as to eliminate or at least reduce the thrust that may otherwise generate on the stump and that would tend to make the stump escape from the socket.

With the airtight hollow space formed between the two membranes, greater insulation is obtained between the stump and the stiff socket wall with a volume that can adjust to the variations of the stump size. Advantageously, the hollow space between the two membranes may be connected to fluid inlet means and to other means for stabilizing pressure within the hollow space. The pressure stabilization means may consist of or comprise one or more suitably calibrated valves. In this way it is possible to maintain a substantially constant pressure inside the hollow space during the prosthesis usage. When the volume, that is, the size of the stump changes due to physiological phenomena, the hollow space volume may change while the same pressure is always maintained on the stump. At the same time, a substantially constant pressure is also ensured between the outer membrane and the inner socket wall, so as to maintain the correct grip between prosthesis and stump. Since the values tolerated by the blood pressure are in the order of 0.2 bar, the prosthesis operating pressure may be comprised within a range of 0.02 and 0.1 bar and preferably between 0.05 and 0.08 bar compared to ambient pressure. Typically, the pressure of 0.2 bar can be tolerated for limited time intervals, whereas a pressure of 0.12 bar can be easily tolerated for a very long time. According to advantageous embodiments of the invention, the operating pressure in the hollow space between the membranes is maintained at around 0.1 bar by the above pressure stabilization means, for example one or more valves calibrated to the pressure to be maintained within the hollow space, and which allow the venting of excess air when pressure tends to increase.

The cap may be associated to a fluid inlet valve, typically air, into the hollow space. The valve may be made to maintain a substantially constant pressure inside the hollow space. As an alternative there may be provided, for example, two valves for the connection to a fluid inlet line in light pressure into the hollow space and one in connection with the external environment for maintaining the desired pressure into the above mentioned hollow space.

In advantageous embodiments it is possible to provide for the inner hollow space of the cap, between the inner membrane and the outer membrane, to be pneumatically connected through at least one conduit for the connection to at least one pump that introduces air into the hollow space. A check valve may be associated to this conduit or to each one of these conduits. The pump may be manual. In particularly advantageous embodiments of the invention, the pump is directly controlled through walking. A dual actuation may also be possible: manual by the user and automatic with walking, using two different pumps with a common conduit or two separate conduits, or a single pump with a dual type of actuation and a single feeding conduit to the hollow space. Moreover, in advantageous embodiments the inner hollow space volume is connected to the outside environment through at least one second conduit and a respective valve calibrated to the pressure value desired inside the hollow space. This calibrated valve makes the excess air pumped into the hollow space be released to the environment, maintaining pressure inside the hollow space always at the same value, as indicated above. In this way, the air pumped through the first conduit always ensures an overpressure in the hollow space, whereas the calibrated valve ensures that this overpressure is always maintained around the desired value, with a sufficient approximation degree. In some embodiments there may be provided multiple venting conduits with respective calibrated valves.

The pneumatic arrangement thus obtained also allows an automatic adjustment to the conditions of the stump whereto the cap is applied. If for any reason the stump undergoes a volume increase, this does not cause an increase in pressure, thanks to the calibrated valve. If the stump undergoes a volume decrease, the prosthesis remains firmly constrained to the same stump, since the potential reduction in pressure is compensated (preferably in an automatic manner by the effect of walking) by the pump.

Further advantageous features of the cap according to the invention are indicated in the annexed claims, which form an integral part of the present description.

As shall be better understood by the description of some embodiments of the invention, with a cap of this kind the anchoring of the prosthesis to the limb stump takes place in a substantially different manner compared to what occurs in current prostheses. In fact, as mentioned above, at present upon the insertion of the limb stump into the concavity of the prosthesis socket, a certain degree of vacuum is generated by means of special sealing members provided in the cap. To this end, the prosthesis may exhibit a venting valve. In substance, when the stump is inserted into the prosthesis seat, a depression is generated between cap and prosthesis, which due to a suction cup effect retains the prosthesis adhering to the stump. The connection thus obtained is precarious on the one side and on the other it does not allow an adjustment of the fixing conditions of the prosthesis to the stump that takes into account the volume variation of tissues over time. Walking may also cause an excessive pressure reduction in the volume between inner socket wall and cap, with consequent negative effects on the stump tissues.

Vice versa, the present invention is based on the idea of generating and stably maintaining a controlled overpressure in the hollow space created inside the cap, wherewith the prosthesis adhesion to the stump is maintained, with a real time adjustment to the actual variable conditions of the tissues of the stump, on the one side preventing excess compression of the tissues and on the other side, preventing the loss of prosthesis control due to an insufficient retaining effect.

The hollow space between the two inner and outer membranes forming the prosthesis cap may take different shapes, optionally also based on the patient's needs. For example, a hollow space may be provided, defined by a single annular volume, or by a plurality of annular volumes spaced from one another by reciprocal welding zones of the membranes, but connected by pneumatic conduits that allow the passage of air or other fluid under pressure, for maintaining all the annular volumes substantially at the same pressure through a single feeding conduit of the pressurized fluid and a single pressure stabilization system.

In other embodiments, the hollow space may be divided into a plurality of non-annular zones, advantageously all interconnected for obtaining an even and controlled pressure in each zone through a single system for pumping and controlling the inner pressure.

In yet further embodiments, there may be provided reciprocal welding zones of the two inner and outer membranes in order to prevent the compression of some predetermined portions of tissue, based on the patient's needs and problems.

It is also possible to make a cap with two or more hollow spaces or portions of hollow space not interconnected to one another, that is, pneumatically separate from each other. In this case, at least one connection conduit to the pump and at least one discharge conduit are suitably provided, with a respective calibrated valve for controlling and stabilizing pressure, for each of said hollow spaces or hollow space portions.

Calibrated valves for pressure control and stabilization are available on the market. They constitute passive elements easy to use and very inexpensive, which at very reduced costs therefore allow providing caps that have an adaptive behaviour upon the variation of usage conditions, allowing a same patient to always have an ideal pressure on the limb stump, even when tissues tend to swell or decrease in volume. Moreover, an easy adjustment of the prosthesis to the patient is possible with a simple pressure stabilization, without needing complex measurement operations for providing a socket perfectly adjusted to the patient, which would then require continuous adaptations also due to the changed conditions of the patient. This allows substantially reducing the costs for making the prosthesis.

Advantageously, the pressure stabilization valve is of a type that allows maintaining the pressure value around the desired value regardless of the valve position or orientation, that is, advantageously, a valve shall be used the behaviour whereof is not influenced by the force of gravity. Valves of this type are available on the market.

According to a different aspect thereof, the invention also relates to a prosthesis, in particular a lower limb prosthesis, comprising a socket associated to a cap of the type described above. The prosthesis may be transfemoral or transtibial. Preferably, the prosthesis is of the endoskeletal type and includes at least one artificial joint, for example the ankle joint in the case of a transtibial prosthesis, or the knee and ankle joints in the case of a transfemoral prosthesis. The socket can be made of any suitable material, substantially stiff, for example synthetic resin optionally reinforced with fibre. Contrary to traditional prostheses, a prosthesis according to the invention, provided with a dual membrane cap of the type described above does not require an adaptation of the stiff portion, that is, of the socket, to the single patient. In fact, the cap adjusts the prosthesis to the patient, without needing expensive and long interventions for adjusting the stiff socket.

Preferably, the artificial joint is associated to a pumping means connected by a conduit to the cap hollow space. The connection may be reversible, that is, a union may be provided between a conduit associated to the actual prosthesis, preferably at least partly made within the socket wall, and a conduit steadily associated to the cap. The aforementioned pressure stabilization valve may be provided along the flow connection between the pump and the inner cap hollow space.

It is also possible for the inflation of the cap hollow space to take place manually by the user with a manual pump. In this case, the user will see to restoring the pressure inside the cap hollow space whenever he/she feels a loosening of the prosthesis grip. The pressure control valve ensures automatic venting in the case of limb swelling and also prevents pressure from increasing excessively by the effect of the manual pumping.

Further features of the prosthesis according to the invention are indicated in the annexed dependent claims, which form an integral part of the present description.

### Brief description of the drawings

The invention will be better understood by following the description and accompanying drawing, which shows a non-limiting practical embodiment of the invention. More in particular, in the drawing:
Fig. 1 shows a perspective view of a socket of a transfemoral prosthesis;
Fig. 2 shows a perspective partly cutaway view of a cap according to the invention to be applied to a stump for the insertion and anchoring within the socket of Fig. 1;
Fig. 2A shows a local section according to II-II of Fig.2;
Fig. 2B shows a local section of the cap of Fig. 2 in a modified embodiment;
Fig. 3 shows an endofemoral prosthesis according to the invention, provided with the cap of Fig. 2 applied to the stump of a lower limb;
Fig. 4 shows a section similar to the section of Fig. 3 in an improved embodiment of the invention;
Fig. 4A shows an enlargement of the separation portion between two zones of the hollow space of the cap of Fig. 4;
Figs. 5A and 5B show diagrams of possible pneumatic circuits for maintaining the calibrated pressure into the hollow space;
Fig. 6 shows a side and partly cutaway view of a cap in a different embodiment; and
Figure 6A shows a local sectional view according to line A-A of Fig. 6.

### Detailed description of embodiments of the invention

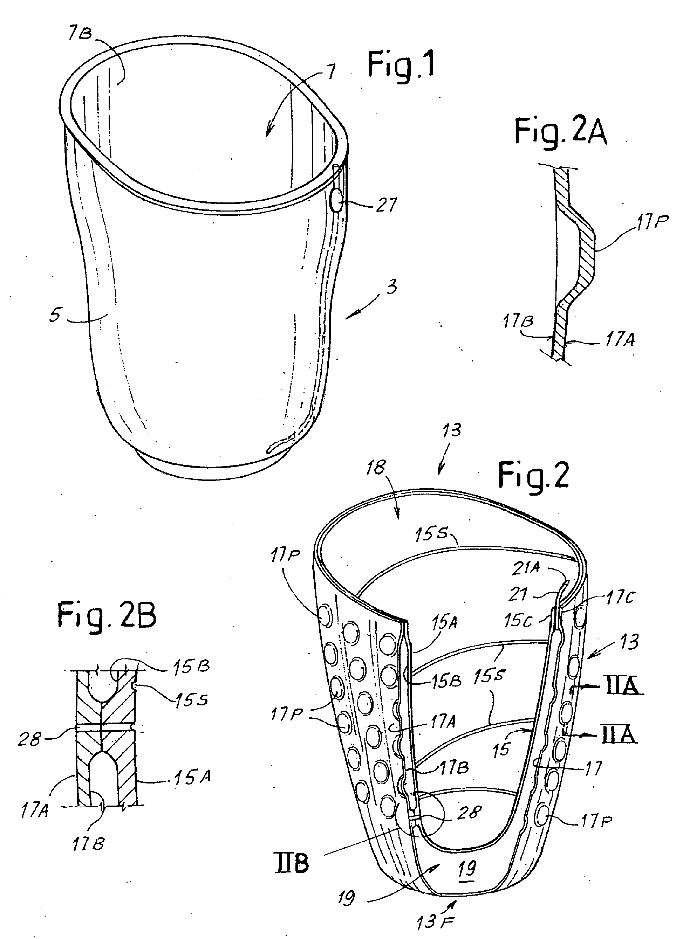

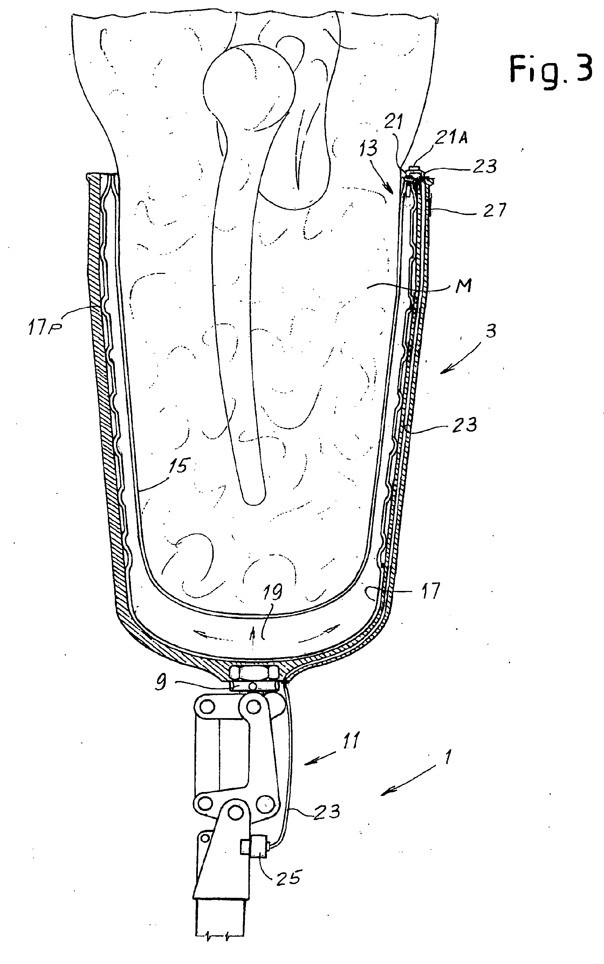

Fig. 1 schematically shows a socket 3 of a transfemoral endoskeletal prosthesis 1. Socket 3 comprises a wall 5 defining an inner space 7, wherein the limb stump is inserted for fixing the prosthesis. At the bottom, socket 3 has a connection 9 to the upper portion of knee 11 which is shown in Fig. 3.

A cap, globally indicated with reference numeral 13 and shown alone in Fig. 2, made of an elastic and flexible material, for example a silicone rubber, is provided to allow the application of the prosthesis to the stump. Cap 13 comprises a first inner membrane 15 and a second outer membrane 17. The inner membrane 15 has an inner surface 15A that defines volume 18 wherein the limb stump is inserted. Reference numeral 15B, on the other hand, indicates the surface of membrane 15 facing outwards, that is, towards the outer membrane 17. The latter in turn has an outer surface 17A intended for contacting the inner surface 7B of space 7 of socket 3 creating an adhesion by the effect of friction between cap 13 and socket 3. The inner surface of the outer membrane 17 is indicated with reference numeral 17B and faces surface 15B of the inner membrane 15. A hollow space 19 is defined between surfaces 15B and 17B. The two upper edges 17C and 15C of the outer membrane 17 and of the inner membrane 15 are welded to one another so that the hollow space 19 is airtight. The welded edges define and surround the inlet of volume 18 for seating stump M.

When stump M of the limb is inserted into volume 18 of cap 13, it is surrounded by the inner membrane 15. This is sized for adhering onto the stump in a comfortable manner, that is, without exerting excessive pressure. Socket 3 of prosthesis 1 (globally shown in Fig. 3) is sized so as to ensure a sufficient degree of comfort to the patient when stump M with the worn cap 13 is positioned within socket 3. The right degree of interference between cap 13 with stump M and socket 3 is ensured by a slight overpressure of the fluid that fills hollow space 19. Typically and preferably, the fluid will simply be ambient air and will be pumped and maintained to the desired pressure in the manner described below. A constant pressure against the inner wall of socket 3 and on stump M, even when the volume of the latter changes, is ensured by a system for controlling the inner pressure of hollow space 19.

In a possible embodiment, cap 13 has a flow passage, provided within the welded zone of edges 17C and 15C and connected to a conduit 21 with an end union 21 A. This union may contain a check valve for allowing air pumping, for example with a manual pump, up to reaching the desired pressure inside hollow space 19 when the patient wears prosthesis 1. This operation needs to be carried out only during the insertion of the stump with cap 13 into socket 3 of prosthesis 1. Once prosthesis 1 has been stabilized to stump M by the effect of the slight pressure into hollow space 19, which makes the outer membrane 17 press against the inner wall of socket 3 and thus retain cap 13 and as a consequence, stump M within socket 3, the patient can walk without losing the prosthesis and without having the stump excessively penetrating within socket 3.

In order to maintain this situation for the entire day, conduit 21 with union 21 A thereof may be connected to a conduit 23 that connects the interior of hollow space 19 to a pumping means, schematically indicated with reference numeral 25, associated to the artificial joint formed by prosthesis 1. In the example shown, since it is a transfemoral prosthesis, the joint is that of the knee. In the case of a transtibial prosthesis, the pumping means may be associated to the ankle joint. A pressure control valve 27 is provided along conduit 23. In other embodiments, valve 27 may be inserted in conduit 21. As shall be explained hereinafter, valve 27 may also be associated to a separate conduit connected to the cap hollow space.

In the substance, valve 27 is calibrated to the pressure (typically 0.1 bar) that is desired into hollow space 19. It constitutes a pressure stabilization means and may also be applied to a different position other than that shown, for example in a conduit separate and independent of conduit 23. Advantageously, valve 27 is of a type that allows maintaining the stabilized pressure to the desired value irrespective of the orientation in the valve space, that is, irrespective of the effect of the force of gravity.

Conduit 23 may advantageously be formed within the thickness of socket 5 of the prosthesis.

The function of pump 25, conduit 23, and valve 27 is to maintain the pressure within the hollow space 19 to a substantially constant value. With the walking movement, pump 25 introduces small amounts of air into hollow space 19, thus making up for any leaks and increasing the volume of hollow space 19 if the stump volume decreases compared to the volume it had when the prosthesis was fit onto the stump. The pressure control valve 27 makes the inside pressure into hollow space 19 not increase neither by the effect of the continuous pumping through pump 25, nor by the effect of a possible increase in the volume of stump M compared to the initial volume.

If there are no leaks in hollow space 19 and if stump M does not change in volume, all the air pumped by pump 25 is released through valve 27, thus maintaining the constant pressure into hollow space 19. If stump M increases in volume, the air released through the pressure control valve 27 will be more than the air pumped by pump 25. Otherwise, if stump M reduces in volume, a part of the air pumped by pump 25 increases the volume of hollow space 19 without being released to the environment.

This prosthesis system formed by prosthesis 1 with socket 3 and by cap 13, provided with pump 25, with the connecting conduits and with hollow space 19, ensures the utmost comfort to the patient, preventing excessive pressure increases on the stump and the consequent serious drawbacks on circulation mentioned above.

At the same time, stump M is prevented from excessively penetrating into socket 3 with consequent interferences between the stump or other body parts and the stiff socket 3.

In order to ensure better comfort to the patient it is advantageously possible to provide for the inner membrane 15 to be less deformable than the outer membrane 17. In this way, the pressure inside the hollow space 19 will tend to deform the outer membrane 17 more, ensuring the adhesion to wall 5 of socket 3, reducing the stress on stump M. This different deformability may be obtained with a different mixture, that is, with a different composition of the material forming membrane 15 compared to that forming membrane 17. As an alternative or in combination, different thicknesses may be used for the two membranes.

According to some embodiments, the outer membrane 17 is provided with protuberances 17P. These protuberances may have any shape. In the embodiment shown (in particular, see Fig. 2A) they have a substantially circular development and a shape about like a cap or a dome, as is seen in particular in the section of Fig. 3. The thickness of membrane 17 may be variable and in particular, the central zone of each protuberance 17P may be thicker than the external zone, wherein protuberance 17P is jointed to the remaining portion of membrane 17. This ensures a high resistance of protuberance 17P in the zone wherein it presses against the inner wall of socket 3. At the same time, the zone with a smaller thickness of each protuberance 17P that surrounds the central zone of the latter ensures a high outwards deformability by the effect of the pressure inside the hollow space 19. Therefore, each protuberance or projection 17P is effectively pressed against the inner surface of wall 5 of socket by the effect of the pressure into the hollow space 19.

In some embodiments (see in particular Figs. 2 and 2B), one or more grooves 15S are provided along the inner surface 15A of the inner membrane 15, which may have a development from the top downwards, that is, from the inlet of cap 13, formed by the welding zone between edges 17C and 15C, towards the bottom of the cap indicated with reference numeral 13F in Fig. 2. These grooves 15S may have, for example, a helical pattern. They serve for collecting the sweat that builds up on the skin of stump M. The sweat can therefore flow gradually along the groove or grooves 15S towards the bottom 13F of cap 13.

In a suitable zone in the lower portion of cap 13, the two membranes 17 and 15 may be welded to each other, as schematically indicated in Fig. 2B. A passage 28 may be made in the welding zone that connects the interior of cap 13 outwards. This passage 28 may be connected with one or more grooves 15S and in this way, the sweat that flows along such grooves can escape from the inner volume of cap 13, that is, from volume 18, outwards, i.e. in the space between the outer surface 17A of membrane 17 and the inner surface 7B of the inner space 7 of socket 3. The presence of protuberances 17P on the outer surface of membrane 17 maintains an adequate empty volume between the cap and the socket, where the sweat may collect and wherefrom it can evaporate. As an alternative, a hole may be provided for discharging sweat in the lower portion of the socket. At least a partial removal of the sweat, and therefore higher comfort for the patient wearing prosthesis 1, are thus obtained.

As is seen in particular in Fig. 3, in some embodiments the hollow space 19 may have a variable size and preferably larger in the lower zone 19A, that is, the zone defined between the bottom of the inner membrane 15 and the bottom of the outer membrane 17. In other words, the distance between the inner membrane 15 and the outer membrane 17 may be larger in the zone wherein most of the compression stress is discharged on the prosthesis. This prevents the squeezing of soft tissues of stump M against the bottom of socket 3, a phenomenon that in traditional prostheses leads to painful consequences for the patient. A dashed, i.e. discontinuous, welding may be made around the lower zone 19A of hollow space 19, between the two membranes, as indicated in Fig. 2B, even when the sweat discharge conduit 28 is not provided. This discontinuous welding allows greater stability between the two membranes and higher comfort and at the same time it allows a distribution with even pressure of the air or other fluid in the entire volume between membranes 15 and 17.

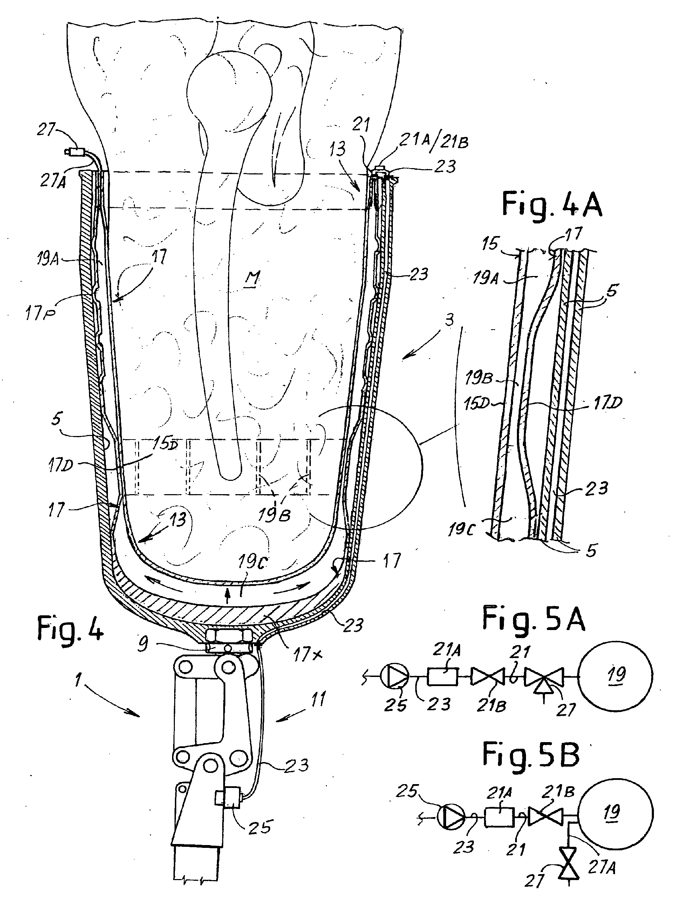

Fig. 4 shows a section similar to that of Fig. 3, wherein same numerals denote same parts or corresponding to those of Fig. 3, of an improved embodiment of the invention. In this embodiment, cap 13 is formed by two membranes, respectively inner 15 and outer 17, connected to one another for example by welding at least along edges 15C and 17C and in at least one intermediate portion 15D, 17D, preferably with an approximately annular pattern. The approximately annular intermediate welding 15D, 17D may be continuous or discontinuous. Preferably, the welding is discontinuous at least by the extent required to set in fluid communication the two chambers, an annular one indicated with reference numeral 19A, and a lower cup-shaped one 19C wherein the hollow space between the two membranes is divided.

The two chambers 19A, 19C are advantageously interconnected pneumatically, that is, in flow communication, so that a single pneumatic circuit of a type similar to that described above may maintain both chambers at a controlled pressure. In the embodiment shown, the pneumatic connection between the two chambers 19A, 19C the hollow space 19 is divided into is obtained through one or more passage gaps 19B made (see the enlargement of Fig. 4A) in the thickness obtained from the reciprocal welding of the two membranes 15, 17 in the annular separation zone between the two chambers. The less advantageous possibility of providing two different separate and not interconnected chambers with two different pneumatic circuits for maintaining the pressure, is not excluded.

A better stabilization of the stump into socket 3 is obtained with such division into two chambers and with the intermediate welding zone.

Advantageously, the outer membrane 17 has a lower zone indicated with reference numeral 17X of larger thickness, for example a thickness from two to five times larger than the remaining membrane portion. This allows obtaining the following advantage. The lower chamber 19C has an outer wall, formed by portion 17X of the outer membrane 17, substantially less deformable than the remaining walls of the hollow space between the two membranes. As a consequence, while a lower chamber 19C is formed, which serves as shock absorber or bearing between the distal end of the stump and the socket bottom, the thrust against the stump that would tend to extract it from the socket and that would otherwise be generated by the deformation due to the expansion of chamber 19C, is reduced or eliminated.

The diagram of Fig. 4 shows a configuration wherein the pressure stabilization valve 27 is separate from the connection conduit to pump 25. In substance, in this case the pneumatic circuit comprises: a pump 25 that is connected through conduit 23 to the hollow space 19 of cap 13 for feeding air into said hollow space; the pressure release and stabilization valve 27, connected to the hollow space 19 through a separate conduit 27A; an optional check valve between the hollow space 19 and conduit 23.

When the cap has an intermediate annular welding between the two membranes 15 and 17, if a sweat removal system is provide as described with reference to Fig. 2B, the sweat discharge hole 28 may advantageously be made at the intermediate annular welding.

Fig. 5A shows a circuit diagram of the pneumatic system associated to cap 13 and to prosthesis 1 for maintaining the pressure inside the hollow space. Reference numeral 19 schematically denotes a closed volume that represents the hollow space 19. This is connected to pump 25 through a conduit 23, a union 21A, a check valve 21B, a conduit 21 and a calibrated valve 27, which releases any overpressure outside. Valve 27 is advantageously calibrated to 0.1 bar, a value to be taken as indicative and non-limiting.

Fig. 5B shows a version of the circuit diagram, corresponding to the embodiment shown in Fig. 4. In this case, the calibrated valve 27 is placed on a different conduit 27A and has the same function described above. Conduit 27A may be directly connected to the inner volume of the hollow space 19, as schematically indicated in Fig. 5B, or it may be a branch of conduit 21. Pump 25 is connected to the inner volume of the hollow space 19 through conduit 23, union 21A and the optional check valve 21B.

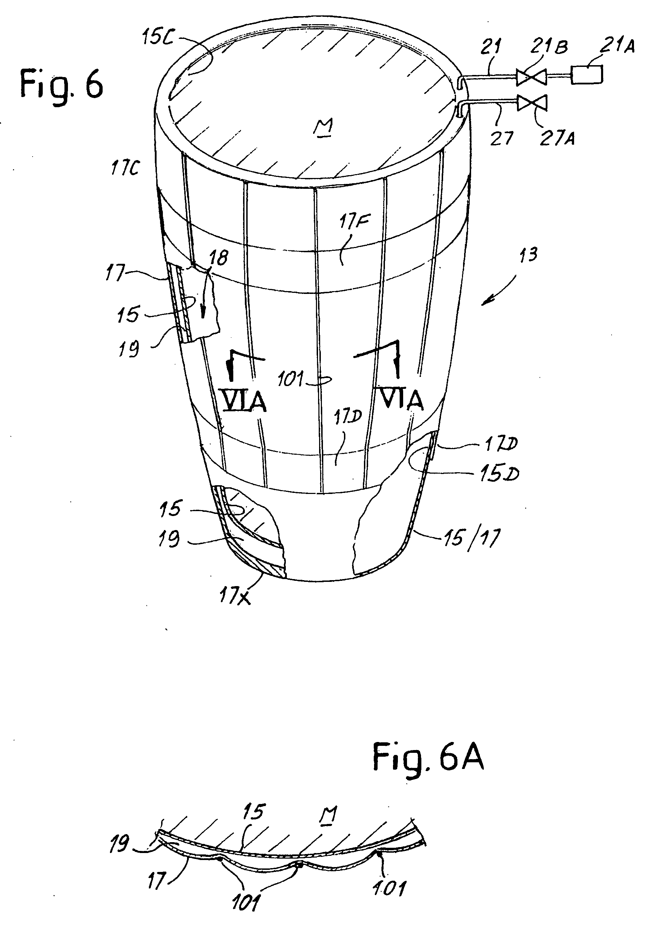

Fig. 6 shows a modified embodiment of cap 13. Same numerals denote same parts or corresponding to those described with reference to the previous figures. Also in this case, cap 13 comprises an inner membrane 15 and an outer membrane 17, whereinbetween an hollow space 19 is formed. The upper edges 15C, 17C of the two membranes 15, 17 are sealingly welded to one another for forming the inner hollow space 19. The conduits 21 and 27A connecting the hollow space 19 to union 21 A for the connection to pump 25 and for the connection to the calibrated valve 27 are arranged at edges 15C, 17C .

At the lower annular zone 17D, 15D, the two membranes 15 and 17 are welded similar to what has been described with reference to Fig. 4. The lower cap portion is sectioned into two zones, right and left in Fig. 4, for showing two different embodiments of the lower zone of cap 13. In the partial local section on the left it is seen that the two membranes 15 and 17 both extend underneath the welding line 17D, 15D for forming a bearing or chamber 19C similar to that shown in Fig. 4. Advantageously, the outer membrane 17 has a bottom portion 17X with larger thickness for the reasons already described.

On the other hand, the section on the right shows a modified embodiment wherein the lower portion of cap 13 is formed by a single membrane, in the example illustrated only membrane 15. As an alternative, only membrane 17 may be provided. The other one of the two membranes ends at the welding line 17D, 15D.

A feature of this embodiment is the presence of substantially inextensible elements with longitudinal development 101 applied on the outer surface of the outer membrane 17. These elements may consist, for example, of portions of threads of a synthetic material, for example Nylon^{®}. The ends of each substantially inextensible element are anchored at welds 17D, 15D and 17C, 15C. Preferably, the intermediate portion of each substantially inextensible element is also fixed to the surface of the outer membrane 17. By substantially inextensible it is meant an element the elongation whereof under the effect of the pressure inside hollow space 19 is negligible compared to the deformation of the material that forms membranes 15 and 17. These membranes may be made of a very thin and therefore highly deformable material. The presence of the substantially inextensible elements makes the outer membrane 17 deform radially outwards under the effect of the air under pressure into hollow space 19, forming a plurality of swellings between each pair of consecutive linear substantially inextensible elements 101, as schematically shown in Fig. 6A. The swellings adhere to the inner wall of socket 3 allowing an adjustment of the cap to the stump and to the socket and generating a friction force on the inner socket wall by the effect of an even small overpressure inside the hollow space 19. This friction force may also be sufficient for preventing the stump from entirely penetrating into the socket, making the presence of the lower chamber 19C unnecessary as in this case the stump remains always spaced from the socket bottom. For this reason, a single membrane 15 or 17 may be provided in the zone underneath welding 17D, 15D. Of course, in this case welding 19B shall be complete and continuous and no air passage gaps or conduits shall be provided in the lower hollow space zone.

It is understood that the drawing shows just one example, provided merely as a practical demonstration of the invention, which can vary in its forms and arrangements, without however departing from the scope of the concept underlying the invention. Any reference numbers in the appended claims are provided to facilitate reading of the claims with reference to the description and to the drawing, and do not limit the scope of protection represented by the claims.

## Claims

1. A cap (13) for the application of a limb prosthesis, in particular a lower limb prosthesis, comprising a dual membrane structure (15, 17), with a first inner membrane (15) and a second outer membrane (17), defining at least one sealed hollow space (19), the inner membrane (15) defining a volume (18) for seating the limb stump (M) and the outer membrane (17) defining a surface of contact and adhesion to the prosthesis socket (3), wherein said hollow space (19) is associated to a pressure stabilization means for stabilizing the inner pressure of said hollow space and an inlet conduit (21) for air under pressure, **characterized in that** said pressure stabilization means comprises a venting valve (27), calibrated to a pressure corresponding to the pressure to maintain within said hollow space (18).

2. Cap according to claim 1, comprising an inlet valve (21B) for air inlet into said sealed hollow space (18).

3. Cap according to claim 1 or 2, wherein said outer membrane (17) and said inner membrane (15) substantially have a bag shape with an external edge (15C, 17C) from which the respective membrane develops defining said volume (18) for seating the limb stump (M) and wherein the external edges of the two membranes are welded to each other.

4. Cap according to claims 2 and 3, wherein said inlet valve (21B) is applied at a thickness of material formed by the reciprocal welding of said two edges (15C, 17C).

5. Cap according to claim 3 or 4, wherein said outer membrane (17) and said inner membrane (15) are connected to each other in at least one zone comprised between said external edges (15C, 17C) and a bottom (13F) of the cap (13).

6. Cap according to claim 5, wherein said outer membrane (17) and said inner membrane (15) are connected to each other along at least one zone comprised between the external edges (15C, 17C) and the bottom (13F) of the cap (13) for forming at least two chambers (19A, 19C), pneumatically connected to each other.

7. Cap according to claim 6, wherein said zone wherein said inner membrane (15) and said outer membrane (17) are connected to each other has a substantially annular pattern.

8. Cap according to one or more of the previous claims, wherein said outer membrane (17) has a bottom (17X) with larger thickness than the remaining membrane portion.

9. Cap according to one or more of the previous claims, wherein the inner membrane (15) has lower deformability than the outer membrane (17).

10. Cap according to one or more of the previous claims, wherein said outer membrane (17) has a plurality of protuberances (17P) defining surfaces of friction with the prosthesis socket (3).

11. Cap according to claim 10, wherein said protuberances (17P) have a variable thickness that increases the protuberance deformability outwards the cap (13) by the effect of the fluid pressure in said hollow space, and wherein each protuberance (17P) preferably has a central zone with larger thickness and an external zone with smaller thickness, whereat the protuberance is jointed to the membrane surrounding zone.

12. Cap according to one or more of the previous claims, comprising a discontinuous or partial welding between the inner membrane (15) and the outer membrane (17) adjacent to a bottom zone (13F) of the cap (13), for delimiting a portion with larger thickness of said hollow space.

13. Cap according to one or more of the previous claims, wherein the inner surface (15A) of the inner membrane (15) has at least one groove (15S) for collecting and conveying sweat and said inner membrane (15) and said outer membrane (17) are welded to one another in at least one area, in the inner portion of the cap, an output channel (28) for the sweat collected by said at least one groove (15S) being made in said area.

14. Cap according to one or more of the previous claims, wherein said inner membrane (15) and said outer membrane (17) are welded to each other along respective edges (15C, 17C) defining an inlet opening of the volume (18) for seating the limb stump (M) and along an intermediate annular welding zone arranged between said edges (15C, 17C) and the bottom of the volume for seating the limb stump (M).

15. Cap according to claim 14, wherein above and underneath said intermediate welding zone there are defined at least two respective chambers (19A, 19C) in flow communication with each other.

16. Cap according to claim 14 or 15, wherein between the welded edges (15C, 17C) of the inner membrane (15) and of the outer membrane (17) and said intermediate annular welding substantially inextensible elements (101) are arranged.

17. Cap according to claim 16, wherein said substantially inextensible elements (101) are thread-like.

18. A limb prosthesis (1), in particular for a lower limb, comprising a socket (3) for the connection to the limb stump (M), a cap (3) according to one or more of the previous claims being seatable in said socket (3), and pumping means (25) for pumping a fluid in the hollow space (19) of said cap (13).

19. Prosthesis according to claim 18, wherein said pumping means (25) are associated to an articulation (11) of said prosthesis so that the walking movement of the patient wearing the prosthesis controls the fluid pumping in said hollow space through said pumping means.

20. Prosthesis according to claim 18 or 19, wherein said socket (3) has a connection conduit (23) between said pumping means (25) and the cap (13).

21. Prosthesis according to claim 20, wherein said connection conduit (23) is made in the thickness of the socket wall.

## Patentansprüche

1. Kappe (13) für die Anwendung einer Gliedmaßenprothese, insbesondere einer unteren Gliedmaßenprothese, mit einer Doppelmembranstruktur (15,17) mit einer ersten inneren Membran (15) und einer zweiten äußeren Membran (17), die zumindest einen Hohlraum (19) definieren, wobei die innere Membran (15) ein Volumen (18) zur Aufnahme des Gliedmaßenstumpfes (M) definiert und die äußere Membran (17) eine Oberfläche des Kontaktes und der Anhaftung des Prothesensockels (3) definiert, wobei der Hohlraum (19) einem Druckstabilisierungsmittel zum Stabilisieren des Innendrucks des Hohlraums und einer Einlassleitung (21) für Druckluft zugeordnet ist, **dadurch gekennzeichnet, dass** das Druckstabilisierungsmittel ein Lüftungsventil (27) aufweist, das auf einen Druck kalibriert ist, der dem Druck entspricht, der innerhalb des Hohlraums (18) zu halten ist.

2. Kappe nach Anspruch 1 mit einem Einlassventil (21B) zum Lufteinlass in den abgedichteten Hohlraum (18).

3. Kappe nach Anspruch 1 oder 2, wobei die äußere Membran (17) und die innere Membran (15) im Wesentlichen eine Taschenform mit einer Außenkante (15C, 17C) besitzen, von der aus die jeweilige Membran sich entwickelt, die das Volumen (18) zur Aufnahme des Gliedmaßenstumpfes (M) definiert und wobei die Außenkante der beiden Membranen miteinander verschweißt sind.

4. Kappe nach Anspruch 2 und 3, wobei das Einlassventil (21B) bei einer Materialdicke angeordnet ist, die durch das gegenseitige Verschweißen der beiden Kanten (15 C, 17 C) gebildet wird.

5. Kappe nach Anspruch 3 oder 4, wobei die äußere Membran (17) und die innere Membran (15) miteinander in zumindest einer Zone verbunden sind, die sich zwischen den Außenkanten (15C, 17C) und einem Boden (13F) der Kappe (13) befindet.

6. Kappe nach Anspruch 5, wobei die äußere Membran (17) und die innere Membran (15) miteinander entlang zumindest einer Zone verbunden sind, die sich zwischen den Außenkanten (15C, 17C) und dem Boden (13F) der Kappe (13) befindet, zum Ausbilden von mindestens zwei Kammern (19A, 19C), die pneumatisch miteinander verbunden sind.

7. Kappe nach Anspruch 6, wobei die Zone, in der die innere Membran (15) und die äußere Membran (17) miteinander verbunden sind, eine im Wesentlichen ringförmige Gestalt aufweist.

8. Kappe nach einem oder mehreren der vorstehenden Ansprüche, wobei die äußere Membran (17) einen Boden (17X) mit einer größeren Dicke als der restliche Membranteil aufweist.

9. Kappe nach einem oder mehreren der vorstehenden Ansprüche, wobei die innere Membran (15) eine geringere Verformbarkeit als die äußere Membran (17) aufweist.

10. Kappe nach einem oder mehreren der vorstehenden Ansprüche, wobei die äußere Membran (17) eine Anzahl von Vorsprüngen (17P) aufweist, die Reibungsflächen mit dem Prothesensockel (3) bilden.

11. Kappe nach Anspruch 10, wobei die Vorsprünge (17P) eine variable Dicke aufweisen, die die Verformbarkeit der Vorsprünge in Richtung nach außen der Kappe (13) durch den Effekt des Fluiddruckes in dem Hohlraum erhöhen, und wobei jeder Vorsprung (17P) vorzugsweise eine Mittenzone mit größerer Dicke und eine Außenzone mit geringerer Dicke aufweist und wobei der Vorsprung mit der die Membran umgebenden Zone verbunden ist.

12. Kappe nach einem oder mehreren der vorstehenden Ansprüche mit einer diskontinuierlichen oder partiellen Schweißung zwischen der inneren Membran (15) und der äußeren Membran (17) angrenzend an eine Bodenzone (13F) der Kappe (13) zum Begrenzen eines Teils des Hohlraums mit größerer Dicke.

13. Kappe nach einem oder mehreren der vorstehenden Ansprüche, wobei die Innenfläche (15A) der inneren Membran (15) mindestens eine Nut (15S) zum Sammeln und Leiten von Schweiß aufweist und wobei die innere Membran (15) und die äußere Membran (17) miteinander in mindestens einem Bereich verschweißt sind, in inneren Teilen der Kappe, wobei ein Auslasskanal (28) für den Schweiß, der in der mindestens einen Nut (15S) gesammelt wird, in dem Bereich ausgebildet ist.

14. Kappe nach einem oder mehreren der vorstehenden Ansprüche, wobei die innere Membran (15) und die äußere Membran (17) miteinander entlang jeweiliger Kanten (15C, 17C) verschweißt sind, die eine Einlassöffnung für das Volumen (18) zur Aufnahme des Gliedmaßenstumpfes (M) bildet, und entlang einer ringförmigen Zwischenschweißzone, die zwischen den Kanten (15C, 17C) und dem Boden des Volumens zur Aufnahme des Gliedmaßenstumpfes (M) angeordnet ist.

15. Kappe nach Anspruch 14, wobei oberhalb und unterhalb der Zwischenschweißzone zumindest zwei jeweilige Kammern (19A, 19C) in Flussverbindung miteinander definiert sind.

16. Kappe nach Anspruch 14 oder 15, wobei zwischen den geschweißten Kanten (15C, 17C) der inneren Membran (15) und der äußeren Membran (17) und der ringförmigen Zwischenschweißung im Wesentlichen nicht ausdehnbare Elemente (101) angeordnet sind.

17. Kappe nach Anspruch 16, wobei die im Wesentlichen nicht ausdehnbaren Elemente (101) fadenförmig sind.

18. Gliedmaßenprothese (1), insbesondere für eine untere Gliedmaße, mit einem Sockel (3) zur Verbindung zu einem Gliedmaßenstumpf (M), einer Kappe (3) nach einem oder mehreren der vorstehenden Ansprüche, die abdichtbar in dem Sockel (3) ist, und Pumpmitteln (25) zum Pumpen eines Fluids in den Hohlraum (19) der Kappe (13).

19. Prothese nach Anspruch 18, wobei die Pumpmittel (25) einem Gelenk (11) der Prothese so zugeordnet sind, dass die Gehbewegung des Patienten, der die Prothese trägt, das Pumpen des Fluids in den Hohlraum durch die Pumpmittel steuert.

20. Prothese nach Anspruch 18 oder 19, wobei der Sockel (3) eine Verbindungsleitung (23) zwischen den Pumpmitteln (25) und der Kappe (13) aufweist.

21. Prothese nach Anspruch 20, wobei die Verbindungsleitung (23) in der Dicke der Sockelwand ausgebildet ist.

## Revendications

1. Un capuchon (13) pour l'application d'une prothèse de membre, en particulier une prothèse de membre inférieur, comprenant un structure de membrane duale (15, 17) avec une première membrane intérieure (15) et une seconde membrane extérieure (17) formant au moins un espace creux étanche (19), la membrane intérieure (15) formant un volume (18) pour loger le moignon de membre (M) et la membrane extérieure (17) formant une surface de contact et d'adhérence à l'enveloppe de prothèse (3), dans lequel ledit espace creux (19) est associé à un moyen de stabilisation de pression pour stabiliser la pression intérieure dudit espace creux et un conduit d'entrée (21) pour l'air sous pression, **caractérisé en ce que** ledit moyen de stabilisation de pression comprend une soupape de ventilation (27), calibrée à une pression correspondant à la pression pour un maintien à l'intérieur dudit espace creux (18).

2. Capuchon selon la revendication 1, comprenant une soupape d'entrée (21B) pour l'entrée d'air dans ledit espace creux étanche (18).

3. Capuchon selon la revendication 1 ou 2, dans lequel ladite membrane extérieure (17) et ladite membrane intérieure (15) ont substantiellement une forme de sac avec un bord externe (15C, 17C) à partir duquel la membrane correspondante se développe en formant ledit volume (18) pour loger ledit moignon de membre (M) et dans lequel les bords externes de deux membranes sont soudés l'un à l'autre.

4. Capuchon selon les revendications 2 et 3, dans lequel ladite soupape d'entrée (21B) est appliquée à une épaisseur de matériau formée par le soudage réciproque desdits deux bords (15C, 17C).

5. Capuchon selon la revendication 3 ou 4, dans lequel ladite membrane extérieure (17) et ladite membrane intérieure (15) sont reliées l'une à l'autre dans au moins une zone compuse entre lesdits bords externes (15C, 17C) et un fond (13F) du capuchon (13).

6. Capuchon selon la revendication 5, dans lequel ladite membrane extérieure (17) et ladite membrane intérieure (15) sont reliées l'une à l'autre le long d'au moins une zone comprise entre les bords extérieurs (15C, 17C) et le fond (13F) du capuchon (13) pour former au moins deux chambres (19A, 19C), reliées de manière pneumatique l'une à l'autre.

7. Capuchon selon la revendication 6, dans lequel ladite zone dans laquelle ladite membrane intérieure (15) et ladite membrane extérieure (17) sont reliées l'une à l'autres a un motif substantiellement annulaire.

8. Capuchon selon une ou plusieurs des revendications précédentes, dans lequel ladite membrane extérieure (17) présente un fond (17X) avec une largeur supérieure à celle du reste de la membrane.

9. Capuchon selon une ou plusieurs des revendications précédentes, dans lequel la membrane intérieure (15) a une déformabilité inférieure à celle de la membrane extérieure (17).

10. Capuchon selon une ou plusieurs des revendications précédentes, dans lequel ladite membrane extérieure (17) comporte une pluralité de protubérances (17P) formant des surfaces de friction avec l'enveloppe de la prothèse (3).

11. Capuchon selon la revendication 10, dans lequel lesdites protubérances (17P) ont une épaisseur variable qui augmente la déformabilité de la protubérance vers l'extérieur du capuchon (13) sous l'effet de la pression du fluide à l'intérieur dudit espace creux et dans lequel chaque protubérance (17P) a de préférence une zone centrale avec un épaisseur supérieure et une zone extérieure avec une épaisseur inférieure, la protubérance étant reliée à la zone entourant la membrane.

12. Capuchon selon une ou plusieurs des revendications précédentes, comprenant un soudage discontinu ou partiel entre la membrane intérieure (15) et la membrane extérieure (17) adjacent à la zone de fond (13F) du capuchon (13), pour délimiter une partie avec une épaisseur supérieure dudit espace creux.

13. Capuchon selon une ou plusieurs des revendications précédentes, dans lequel ladite surface intérieure (15A) de la membrane intérieure (15) comporte au moins une rainure (15S) pour collecter et convoyer la sueur et ladite membrane intérieure (15) et ladite membrane extérieure (17) sont soudées l'une à l'autre à au moins un endroit, dans la partie intérieure du capuchon, un canal de sortie (28) étant créé audit endroit pour la sueur collectée par ladite ou lesdites rainure(s) (15S).

14. Capuchon selon une ou plusieurs des revendications précédentes, dans lequel ladite membrane intérieure (15) et ladite membrane extérieure (17) sont soudées l'une à l'autre le long de bords respectifs (15C, 17C) formant une ouverture d'entrée du volume (18) pour loger le moignon de membre (M) et le long d'une zone de soudage annulaire intermédiaire disposée entre lesdits bords (15C, 17C) et le fond du volume pour loger le moignon de membre (M).

15. Capuchon selon la revendication 14, dans lequel au-dessus et en dessous de ladite zone de soudage intermédiaire sont formées au moins deux chambres respectives (19A, 19C) en communication fluidique l'une avec l'autre.

16. Capuchon selon la revendication 14 ou 15, dans lequel entre les bords soudés (15C, 17C) de la membrane intérieure (15) et de la membrane extérieure (17) et ledit soudage annulaire intermédiaire, des éléments substantiellement non extensibles (101) sont agencés.

17. Capuchon selon la revendication 16, dans lequel lesdits éléments substantiellement non extensibles (101) sont en forme de fil.

18. Prothèse de membre (1), en particulier pour un membre inférieur, comprenant une enveloppe (3) pour la liaison au moignon de membre (M), un capuchon (3) selon une ou plusieurs des revendications précédentes pouvant être logé dans ladite enveloppe (3) et des moyens de pompage (25) pour pomper un fluide dans l'espace creux (19) dudit capuchon (13).

19. Prothèse selon la revendication 18, dans laquelle lesdits moyens de pompage (25) sont associés à une articulation (11) de ladite prothèse de sorte que le mouvement de marche du patient portant la prothèse commande le pompage de fluide dans ledit espace creux par l'intermédiaire desdits moyens de pompage.

20. Prothèse selon la revendication 18 ou 19, dans laquelle ladite enveloppe (3) comporte un conduit de liaison (23) entre lesdits moyens de pompage (25) et le capuchon (13).

21. Prothèse selon la revendication 20, dans laquelle ledit conduit de liaison (23) est réalisé dans l'épaisseur de la paroi de l'enveloppe.
